(19) Europäisches Patentamt
European Patent Office
Office européen des brevets

(11) **EP 3 143 395 B1**

(12) **FASCICULE DE BREVET EUROPEEN**

(45) Date de publication et mention de la délivrance du brevet:
**20.07.2022  Bulletin 2022/29**

(21) Numéro de dépôt: **15720118.7**

(22) Date de dépôt: **25.03.2015**

(51) Classification Internationale des Brevets (IPC):
**G01N 33/04** *(2006.01)*   **G01N 21/64** *(2006.01)*
**G01N 21/49** *(2006.01)*   **G01N 15/06** *(2006.01)*
**G01N 21/82** *(2006.01)*   G01N 15/00 *(2006.01)*

(52) Classification Coopérative des Brevets (CPC):
**G01N 21/49; G01N 15/06; G01N 21/6486;
G01N 21/82; G01N 33/04;** G01N 2015/0065;
G01N 2015/0693

(86) Numéro de dépôt international:
**PCT/IB2015/052196**

(87) Numéro de publication internationale:
**WO 2015/150982 (08.10.2015 Gazette 2015/40)**

(54) **PROCÉDÉ D'ESTIMATION DES PROTÉINES SÉRIQUES DÉNATURÉES DANS UN LAIT**

VERFAHREN ZUR SCHÄTZUNG VON DENATURIERTEN SERUMPROTEINEN IN MILCH

METHOD FOR ESTIMATING DENATURED SERUM PROTEINS IN MILK

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **31.03.2014  FR 1452818**

(43) Date de publication de la demande:
**22.03.2017  Bulletin 2017/12**

(73) Titulaire: **Spectralys Innovation
93230 Romainville (FR)**

(72) Inventeurs:
• **BIRLOUEZ, Inès
  95120 Ermont (FR)**
• **LACOTTE, Pierre
  34500 Beziers (FR)**
• **ACHARID, Abdelhaq
  95400 Arnouville (FR)**

(74) Mandataire: **Gevers & Orès
Immeuble le Palatin 2
3 Cours du Triangle
CS 80165
92939 Paris La Défense Cedex (FR)**

(56) Documents cités:
EP-A1- 0 922 221     DE-A1- 2 103 137
DE-A1- 4 218 555     DE-B1- 2 816 229
US-A- 3 841 834

• ANDREY BOGOMOLOV ET AL: "Quantitative determination of fat and total protein in milk based on visible light scatter", FOOD CHEMISTRY, ELSEVIER LTD, NL, vol. 134, no. 1, 14 février 2012 (2012-02-14), pages 412-418, XP028416940, ISSN: 0308-8146, DOI: 10.1016/J.FOODCHEM.2012.02.077 [extrait le 2012-02-22]
• ALISA LAMB ET AL: "Optical backscatter method for determining thermal denaturation of [beta]-lactoglobulin and other whey proteins in milk", JOURNAL OF DAIRY SCIENCE, vol. 96, no. 3, mars 2013 (2013-03), pages 1356-1365, XP055133520, ISSN: 0022-0302, DOI: 10.3168/jds.2012-5791

**Description**

L'invention porte sur un procédé d'estimation des protéines sériques dénaturées dans un lait Ce procédé s'applique en particulier à l'industrie laitière, et notamment à la fabrication du fromage, des yaourts, des laits en poudre, des poudres infantiles (lait en poudre pour nourrissons) et autres produits laitiers.

On entend par « protéine dénaturée » une protéine qui a perdu sa structure tridimensionnelle secondaire et tertiaire d'origine, pour former des agrégats,

Ces agrégats sont de taille et de nature diverse, selon qu'il y a ou non des caséines dans le milieu. En présence de caséines, les protéines sériques s'agrègent par pont disulfure avec les caséines, notamment avec la caséine cappa. En l'absence de caséines ou si les caséines sont insuffisantes au regard des protéines sériques en cours de dénaturation, la totalité ou une part de ces protéines s'agrège sur elle-même.

La mesure des protéines sériques dénaturées au cours des différents traitements, en particulier thermiques (U.H.T, pasteurisation), auquel est soumis un lait ou dérivé, est fondamentale pour apprécier la qualité technologique du lait, pour standardiser cette qualité à un niveau optimal en terme de compromis entre rendement ou rentabilité et qualité, ainsi que pour prédire la qualité du produit fini, comprise comme le niveau de texture (fromage, yaourt) ou la faculté de resolubilisation (poudre de lait et poudre infantile) du produit

C'est le cas de la technologie fromagère, par exemple, où le taux de dénaturation des protéines sériques (rapport entre les protéines sériques dénaturées et les caséines) conditionne le rendement fromager. En effet, les protéines dénaturées coaguleront avec les caséines alors que les protéines sériques natives s'écouleront du camé.

C'est pourquoi les industriels fromagers utilisent souvent un traitement thermique de leur lait, voire un enrichissement de ce lait avec des caséines et des rétentats de sérums dénaturés, pour augmenter le taux de protéines du lait qui sera retenu dans le caillé lors de la coagulation.

Cependant, un taux trop élevé de protéines sériques dénaturées risque de compromettre la vitesse et qualité de la coagulation, entraînant une chute des cadences et une perte de qualité du fromage, notamment en texture et goût.

Des problématiques similaires se posent pour la fabrication des yaourts, des poudres de lait et sérum et des poudres infantiles.

Dans le cas des yaourts, la forme de dénaturation des protéines sériques, sur les caséines ou sur elles-mêmes, résulte en une faille très différente des agrégats formés par les protéines sériques ce qui a un impact important sur la rhéologie et donc la texture du produit.

Enfin, dans le cas des formules infantiles, la taille des agrégats va générer des problèmes de resolubillsation dans le biberon.

Aujourd'hui, aucune méthode rapide n'existe pour doser les protéines dénaturées dans un lait qui a subi un traitement thermique (U.H.T, pasteurisation) ou un mélange de laits et/ou dérivés du lait, type lait fromager, ou encore des poudres de lait,

Une technique de laboratoire permettant un dosage des protéines solubles est la chromatographie.

On peut notamment citer l'article de V. Bonfatti et al. « Validation of a new reversed-phase high-performance liquid chromatography method for separation and quantification of bovine milk protein genetic variants » et celui de G. Bobe et al. « Séparation and quantification of bovine milk proteins by reversed-phase high-performance liquid chrornatography ».

Ces deux documents montrent qu'il est possible de doser d'une part, l'ensemble des protéines d'un lait, qui comprend les caséines et les protéines sériques, et d'autre part, les seules protéines sériques non dénaturées.

Le dosage de l'ensemble des protéines est réalisé grâce à une solubilisation des micelles de caséine et des protéines sériques éventuellement dénaturées dans un tampon adéquat dénaturant, alors que les protéines sériques non dénaturées sont toujours solubles.

Par ailleurs, les protéines sériques non dénaturées sont dosées grâce à une précipitation des caséines et des protéines sériques dénaturées dans un tampon adéquat à pH 4,6.

Par différence entre les protéines sériques natives et les protéines sériques totales, dosées indépendamment, on peut déterminer la teneur en protéines sériques dénaturées.

Cependant, la chromatographie est trop complexe, longue et coûteuse à mettre en œuvre pour une application industrielle.

En effet, cette méthode nécessite deux dosages indépendants pour pouvoir déterminer la quantité de protéines sériques dénaturées.

De plus, ce double dosage entraîne un doublement de l'erreur sur la teneur en protéines sériques dénaturées.

Une autre technique connue est décrite dans l'article de I. Recio et al. « Détermination of denatured serum proteins in the casein fraction of heai-treated milk by capillary zone efectrophoresis ».

Elle consiste à traiter thermiquement le lait à pH 4,6 pour faire précipiter les caséines et les protéines dénaturées, puis à solubiliser ce précipité. Par électrophorèse capillaire, on mesure directement la teneur en protéines sériques dénaturées et la teneur en caséines.

La chromatographie liquide haute pression classique décrite plus haut peut également être utilisée mais la séparation est rendue difficile par des problèmes de colmatage de la colonne de chromatographie, malgré la préparation préalable des échantillons pour les resolubiliser après précipitation à pH 4,6.

L'électrophorèse capillaire constitue une technique intéressante car elle n'utilise pas de colonne, Il n'y a donc pas de risque de colmatage,

Cependant, cette technique ne convient pas à des applications industrielles car elle met en œuvre des appareils complexes, d'un coût élevé et nécessite un savoir-faire important.

Il est également possible de doser les protéines sériques totales et solubles avant et après traitement thermique par la méthode de Kjeldahl qui constitue la méthode de référence.

Cette méthode est définie dans la norme ISO 8688. Elle est basée sur une minéralisation de l'échantillon et le dosage calorimétrique de l'azote.

Elle peut être appliquée sur un premier échantillon de lait cru pour déterminer les protéines totales (caséines et protéines sériques solubles). Elle peut également être utilisée sur ce même échantillon de lait cru, après précipitation des caséines à pH 4,6, pour déterminer les protéines sériques solubles. On en déduit par différence la teneur en caséines.

Un deuxième échantillon, du même lait cru, est traité thermiquement La méthode est appliquée sur cet échantillon, après précipitation des caséines et des protéines sériques dénaturées à pH 4,6, ce qui permet d'obtenir le taux de protéines sériques demeurées solubles. Par différence, on calcule donc le taux de protéines sériques dénaturées au cours du traitement thermique.

Ces trois dosages permettent de déterminer le, ratio entre les protéines sériques dénaturées et les caséines.

Cependant, cela nécessite de prélever des échantillons à deux étapes distinctes du procédé de traitement, ce qui est peu pratique en milieu industriel. Par ailleurs, la méthode de Kjeldahl doit être mise en œuvre en laboratoire. Elle utilise des produits chimiques dangereux et polluants et nécessite plusieurs heures.

On peut encore noter que le résultat est obtenu par une succession de différences entre deux mesures, ce qui entraîne une erreur importante sur le résultat final, l'erreur liée à une mesure se cumulant avec celle liée à une autre mesure.

De plus, cette méthode ne permet pas toujours de fournir le ratio entre les protéines sériques dénaturées et la caséine, très utile pour les industriels.

En effet, on n'est jamais certain qu'un lait cru l'est vraiment et donc qu'il n'y a aucune trace de protéines dénaturées sur les caséines.

Il est donc difficile de déterminer avec certitude le taux de caséines par la différence entre protéines totales et protéines solubles.

Le document US 3,841,834 divulgue un procédé pour mesurer automatiquement la teneur totale en protéines contenues dans un échantillon de lait, sans permettre d'estimer la teneur en protéines sériques dénaturées.

Le document Bogomolov et al. Food Chemistry 134 (2012) 412-418 divulgue un procédé de détermination du taux de protéines total et du taux de matières grasses total d'un échantillon de lait non transparisé.

Le document Lamb et al., J. Dairy sci. 96 :1356-1365 divulgue un procédé permettant de suivre en temps réel l'évolution de l'agrégation des protéines sériques dénaturées et des caséines dans un échantillon de lait pur soumis à un traitement thermique.

L'invention vise à remédier aux inconvénients précités de l'art antérieur, et plus précisément à procurer une méthode de dosage des protéines dénaturées dans un lait, pouvant être mise en œuvre de manière simple et rapide (en quelques minutes) même en milieu industriel.

Ici, on entend par « lait » aussi bien le lait animal (généralement de vache, brebis ou chèvre), entier, partiellement ou totalement écrémé, destiné à la consommation directe - éventuellement après thermisation, pasteurisation, stérilisation, concentration, séchage ou ultra/microfiltration - que les laits diversement traités, mélangés et éventuellement enrichis de protéines etc., destinés à diverses transformations industrielles (fabrication du fromage, du yaourt, du lait concentré ou en poudre...), ainsi que les laits « reconstitués » à partir de poudres de lait ou poudres infantiles (laits pour nourrissons en poudre, contenant également des additifs tels que des huiles, divers glucides, vitamines et minéraux).

Dans le cadre de l'invention, on entend également par lait, un lait fromager résultant du mélange de divers ingrédients laitiers, ou encore un lait sans caséines, soit un sérum tel que produit par microfiltration du lait (filtrat) ou par égouttage d'un caillé, puis éventuellement séché après divers autres traitements (déminéralisation, concentration ... ).

Conformément à l'invention, un tel résultat est atteint par un procédé d'estimation des protéines sériques dénaturées dans un lait ayant subi un traitement thermique, le procédé comportant les étapes successives consistant à :

a) rendre transparent un échantillon dudit lait,
b) mesurer la diffusion dans ledit échantillon transparisé pour en déduire un premier indice PSD représentatif de la teneur en protéines sériques dénaturées.

En effet, dans le cadre de l'invention, il a été mis en évidence qu'une mesure de diffusion, du type Rayleigh, du lait rendu transparent permet d'estimer avec une bonne précision la teneur en protéines dénaturées.

Ainsi, les inventeurs ont pu établir que la teneur en protéines dénaturées dans un échantillon de lait comportant peu de matières grasses était proportionnelle à la valeur de la diffusion dans cet échantillon.

Ceci pourrait s'expliquer par l'influence des agrégats de protéines dénaturées sur la diffusion de Rayleigh ou de Mie, selon la taille de ces agrégats. La concentration plus importante de ces agrégats viendrait augmenter la diffusion de l'échantillon.

De préférence, lorsque le lait comporte des matières grasses, le procédé comprend, après l'étape (a), les étapes complémentaires suivantes consistant à :

(c) déterminer un deuxième indice PT représentatif de la quantité totale de protéines dans l'échantillon transparisé à l'étape (a),

(d) à provoquer la précipitation des caséines et des protéines sériques dénaturées dans ufi autre échantillon dudit lait, de manière à obtenir un précipité et un surnageant,

(e) déterminer un troisième indice PSS représentatif de la teneur dudit surnageant en protéines sériques solubles,

(f) déterminer un quatrième indice DP représentatif de la dénaturation des protéines dans ledit surnageant, et

(h) estimer la teneur en protéines sériques dénaturées à partir desdits premier, deuxième, troisième et quatrième indices.

L'invention concerne également un procédé d'estimation du ratio protéines sériques dénaturées / caséines dans un lait ayant subi un traitement thermique, le procédé comportant les étapes consistant à :

(a) rendre transparent un échantilion dudit lait,

(b) mesurer la diffusion dans ledit échantillon transparisé pour en déduire un premier indice PSD représentatif de la teneur en protéines sériques dénaturées,

(c) déterminer un deuxième indice PT représentatif de la quantité totale de protéines dans l'échantillon fransparisé à l'étape (a),

(d) à provoquer la précipitation des caséines et des protéines sériques dénaturées dans un autre échantillon dudit lait, de manière à obtenir un précipité et un surnageant,

(e) déterminer un troisième indice PSS représentatif de la teneur dudit surnageant en protéines sériques solubles,

(f) déterminer un quatrième indice DP représentatif de la dénaturation des protéines dans ledit sumageant et

(i) estimer le ratio protéines sériques dénaturées/caséines à partir desdits premier, deuxième, troisième et quatrième indices.

De façon préférée, les procédés selon l'invention comprennent, après l'étape (d), une étape complémentaire (g) consistant à déterminer un cinquième indice FAST représentatif d'un niveau de traitement thermique subi par ledit échantillon.

De façon avantageuse, au moins une desdites étapes (a) à (g) met en œuvre une mesure optique pour déterminer l'indice correspondant.

Dans l'étape (c), le deuxième indice PT peut être déterminé par une mesure de fluorescence du tryptophane dans l'échantillon transparisé.

Dans l'étape (d), la précipitation des protéines peut être obtenue en ajoutant à un autre échantillon dudit lait un tampon de force ionique et de pH appropriés.

Dans l'étape (e), le troisième indice PSS peut être obtenu par une mesure de la fluorescence du tryptophane dans le surnageant transparent obtenu à l'étape (d).

Dans l'étape (f), le quatrième indice DP peut être obtenu par une mesure de la diffusion dans le surnageant transparent obtenu à l'étape (d).

Le cinquième indice FAST peut être représentatif d'une teneur du surnageant obtenu à l'étape (d) en produits de Maillard.

Dans l'étape (g), le cinquième indice FAST peut être obtenu par une mesure de fluorescence desdits produits de Maillard.

Enfin, chacune des étapes (h) et (i) est avantageusement mise en œuvre par régression multilinéaire,

L'invention sera mieux comprise et d'autres buts, avantages et caractéristiques de celle-ci apparaîtront plus clairement à la lecture de la description qui suit et qui est faite au regard des dessins annexés sur lesquels :

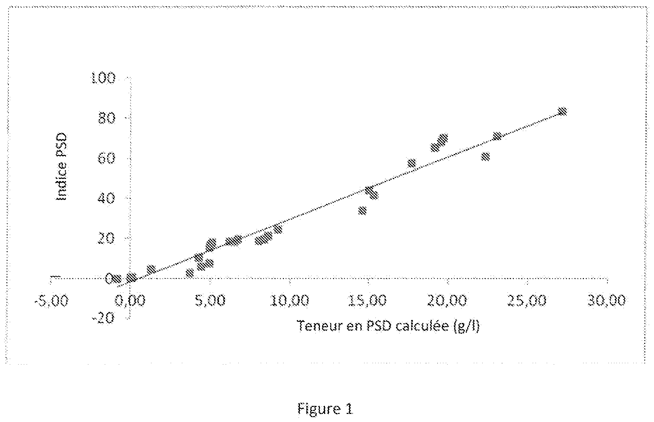

- la figure 1 est un graphique illustrant la corrélation existant entre les teneurs en protéines dénaturées de différents faits modèles ne comportant pas de matière grasse, calculées à partir des constituants des laits modèles (en abscisses) et les valeurs de l'indice PSD déterminé par les étapes (a) et (b) du procédé selon l'invention (en ordonnées),

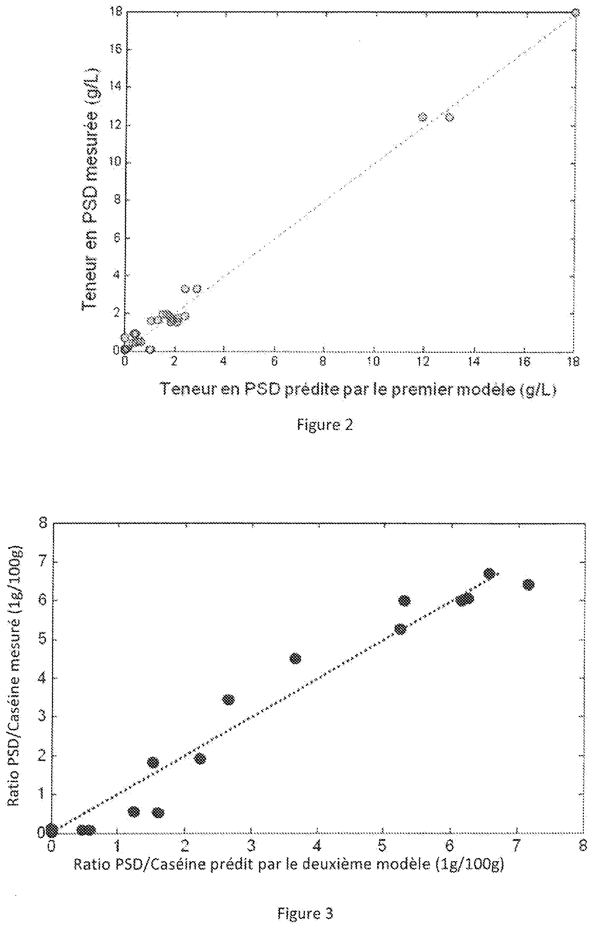

- la figure 2 est un graphique illustrant la régression entre les teneurs en protéines sériques dénaturées (PSD) mesurées par électrophorèse capillaire (en ordonnées) et prédites par les étapes (a) à (g) du procédé selon l'invention (en abscisses) pour différents laits fromagers industriels comportant des matières grasses,
- la figure 3 est un graphique illustrant la régression entre les teneurs en protéines sériques dénaturées (PSD) en pourcentage par rapport à la teneur en caséines dans ces mêmes laits fromagers industriels, mesurées par électrophorèse capillaire (en ordonnées) et prédites par les étapes (a) à (g) du procédé selon l'invention (en abscisses).

Les procédés selon l'invention vont maintenant être décrits de façon détaillée,

L'étape (a) du procédé consiste à rendre transparent un échantillon de lait à analyser.

On connait de nombreuses méthodes pour rendre le lait transparent. Les articles mentionnés plus haut décrivent de telles méthodes.

Elles consistent à diluer l'échantillon de lait dans une proportion de 500 à 1 000, avec une solution qui permet de solubiliser la matière grasse par un solvant organique non dénaturant pour les protéines, puis les caséines grâce à un chéiateur de calcium qui maintient des ponts entre les peptides de caséines et des agents antioxydants qui rompent les liaisons disulfure et, enfin, grâce à un surfactant qui permet de solubiliser des protéines hydrophobes (micelles de caséines et protéines sériques dénaturées).

Ainsi, un tampon, par exemple de phosphate de sodium ou de potassium, contenant des sels de citrate ainsi qu'un surfactant de type sodium dodécylsulfate ou urée permet de chéiater le calcium, de rompre la micelle de caséine et de solubiliser en partie les globules gras (voir l'article de L Recio et al. cité plus haut ou encore l'article de Fox et al., Journal of Dairy Science, Volume 46, issue 4, April 1963, Pages 302-309), comme le mélange proposé par l'article de Bobe et al. cité précédemment qui contient du GdnHcl (hydrochlorure de guanidine) et un solvant.

D'autres tampons peuvent être envisagés, par exemple un tampon simple comprenant des sels de phosphate à pH 6,8 et de l'EDTA. Un tel tampon permet de rendre le lait transparent après dilution, mais à un niveau inférieur à celui obtenu avec les deux exemples mentionnés ci-dessus.

L'étape (b) de mesure de la diffusion consiste à mesurer les photons réfléchis par l'échantillon de lait transparent qui a préalablement été excité par ces mêmes photons.

On rappelle que la diffusion (dénommée diffusion de Rayleigh, lorsque la dimension des particules de la matrice est proche de la longueur d'onde d'excitation, ou diffusion de Mie, lorsque celle-ci est très au-dessus de la longueur d'onde), correspond à une interaction élastique (sans perte d'énergie) entre la lumière à la longueur d'onde considéré et la matrice. Cette interaction rend compte de la structure de la matrice et notamment de la taille et de la concentration des particules de dimension proche de la longueur d'onde.

Différents ouvrages expliquent ce phénomène complexe et difficilement quantifiable dans des matrices hétérogènes complexes :

- C.F. Bohren and D,R. Huffman. Absorption and scattering of light by small particles. John Wiley & Sons, New York, 1983,
- Light scattering by small particles. By H. C. van de Hulst. New York (John Wiley and Sons), London (Chapman and Hall), 1957,
- Wave Propagation and Scattering in Random Media, Akira Ishimaru, January 1999, Wiley-IEEE Press.

Ainsi, la mesure de la diffusion d'une solution simple de protéines sériques est un indice direct et proportionnel de la concentration en protéines dénaturées (indice PSD), du fait de la formation d'agrégats de protéines dénaturées de taille beaucoup plus importante que celle des protéines unitaires solubles. La diffusion en mode conventionnel peut-être utilisée sur une large gamme du proche au lointain UV, y compris dans le visible. Cependant, la sensibilité est plus importante dans l'UV.

Cette étape (a) de transparisation est tout d'abord réalisée sur des laits ne comportant pas de matières grasses.

De façon plus spécifique, les laits utilisés sont des laits modèles reconstitués en laboratoire par association de poudre de sérum, de lait en poudre et d'eau, ce lait reconstitué ayant subi un traitement thermique. En pratique, plusieurs laits modèles ont été préparés dans lesquels les teneurs en sérum et en lait en poudre sont variables pour obtenir des valeurs différentes de la teneur en protéines sériques totales et du ratio entre les protéines sériques totales et les caséines. Le traitement thermique permet de produire des protéines sériques dénaturées en concentrations variables,

La figure 1 est un graphique illustrant la corrélation existant entre d'une part, les teneurs en protéines dénaturées des différents laits modèles calculées, pour chaque lait modèle, par différence entre les protéines sériques totales mises en œuvre dans le lait modèle et la teneur en protéines sériques solubles après traitement thermique, dosée par la méthode Kjeidahl (en abscisses) et d'autre part, les valeurs de l'indice PSD déterminées par les étapes (a) et (b) du procédé selon l'invention (en ordonnées).

La figure 1 montre qu'une excellente régression est observée entre la mesure de diffusion en mode conventionnel (indice PSD), représentative de la teneur en protéines sériques dénaturées (PSD), et celle calculée directement pour les laits modèles, comme indiqué plus haut.

Le coefficient de corrélation est de 0,98.

La précision obtenue est d'autant meilleure que le lait comporte peu de matières grasses.

En effet, la taille des globules gras modifie la diffusion et, par voie de conséquence, la relation entre la concentration des agrégats de protéines dénaturées et la diffusion.

il a été établi que d'autres mesures permettaient d'estimer la teneur en protéines sériques dénaturées, même en présence d'un lait comportant des matières grasses,

Ces différentes mesures vont maintenant être décrites.

Une mesure complémentaire a pour objet de déterminer un deuxième indice PT représentatif de la quantité totale de protéines dans l'échantillon transpansé. Cette détermination peut être réalisée par une mesure de la fluorescence du tryptophane dans l'échantillon transparisé, consistant en une étape (c) qui est réalisée après l'étape (a).

Cette mesure de la fluorescence du tryptophane peut être réalisée par la méthode décrite dans l'article de Fox et al. cité plus haut.

Le principe de cette méthode est de considérer que, si le milieu est transparent, s'applique la loi de Beer Lambert qui relie quantitativement la fluorescence d'une molécule, ici le tryptophane (Trp), et la concentration en protéines porteuses de cet acide aminé.

Dans le cas du lait, toutes les protéines contiennent du tryptophane, si bien que cette méthode permet le dosage des protéines totales contenues dans l'échantillon.

Cette mesure permet d'estimer la quantité totale de protéines dans l'échantillon transparisé.

Une autre mesure complémentaire a pour objet de déterminer un troisième indice PSS représentatif de la teneur en protéines sériques solubles.

Cette mesure est réalisée grâce aux étapes suivantes consistant :

(d) à préparer un autre échantillon dudit lait auquel est ajouté un tampon de force ionique et de pH appropriés pour obtenir d'une part, un précipité de caséines et de protéines dénaturées par ledit traitement thermique et d'autre part, un surnageant transparent qui renferme des protéines sériques encore solubles.
(e) à doser le tryptophane présent dans ledit surnageant transparent.

Les deux étapes (d) et (e) sont décrites et illustrées dans le document EP-0 922 221.

De préférence, le tryptophane présent dans le surnageant est dosé par sa fluorescence; par exemple, on effectue la mesure à une longueur d'onde d'excitation de 290 nm et à une longueur d'onde d'émission comprise entre 330 et 350 nm pour obtenir un maximum de sensibilité. Le dosage du tryptophane pourrait être également effectué par son absorption U.V.

Le document EP-0 922 221 a établi que la fluorescence du tryptophane du surnageant transparent est une mesure rapide de la concentration en protéines solubles faiblement dénaturées et qu'elle est très fortement corrélée au dosage des protéines (Kjeldahl ou méthode calorimétrique du Biuret; r=0,99).

Les étapes (d) et (e) permettent donc d'estimer la quantité de protéines sériques solubles (ou non dénaturées) dans le surnageant.

Une autre mesure complémentaire consiste à déterminer un quatrième indice représentatif de la dénaturation des protéines dans le surnageant obtenu à l'étape (d).

Cette détermination est réalisée par une mesure de diffusion dans le surnageant, correspondant à une étape (f).

Cette étape (f) donne une indication sur la dénaturation des protéines. En effet, elle rend compte de la taille moyenne des particules protéiques dans le surnageant de protéines sériques solubles. Même si ces protéines ne sont pas encore rendues insolubles, elles ont subi au cours du traitement thermique une première modification de structure et ont notamment formé des petits agrégats visibles au travers de la diffusion.

Elle est réalisée sur le surnageant obtenu à l'étape (d).

il s'agit d'une mesure de la diffusion à 280 nm (pour une excitation à 280 nm).

Les étapes (a) à (f) ont été décrites successivement. Cependant, les étapes (a), (b) et (c) d'une part, et (d), (e) et (f) d'autre part peuvent être réalisées en parallèle ou encore dans un ordre quelconque.

Une dernière mesure complémentaire a pour objet de déterminer un cinquième indice FAST représentatif de la réaction de Maillard sur les protéines du surnageant, obtenu à l'étape (d), réaction fortement dépendante de la charge thermique appliquée au lait.

Elle peut consister à doser les sous-produits fluorescents issus de la réaction avancée de Maillard présents dans ledit surnageant.

Cette étape complémentaire (g) permet donc de déterminer un indice thermique global qui renseigne sur le type de traitement thermique subi par le lait et la qualité de ce traitement.

Elle est décrite et illustrée dans le document EP-0 922 221.

Cet indice renseigne sur la charge thermique absorbée par le lait et donc sur la dénaturation thermique des protéines sériques pour déterminer la teneur en protéines sériques solubles.

Cette étape (g) peut être réalisée après l'étape (d) en parallèle des étapes (e) et (f), ou encore avant ou après ces étapes (e) et (f).

La figure 2 est un graphique illustrant la régression entre la teneur en protéines sériques dénaturées (PSD) mesurée par électrophorèse capillaire et prédite par les étapes (a) à (g) du procédé selon l'invention.

Les mesures d'électrophorèse capillaire et les étapes (a) à (f) du procédé selon l'invention ont été réalisées sur des échantillons fournis par des partenaires industriels et correspondant à des laits fromagers industriels, comprenant du lait écrémé, du retentât de microfiltration enrichi en caséines, éventuellement du retentât de sérum chauffé pour permettre sa dénaturation, de la matière grasse laitière, et enfin un mélange de sels minéraux, notamment du calcium.

Ainsi, d'un côté, les protéines du lait ont été analysées par la technique décrite par Recio et Olieman : après solubilisation des caséines et protéines dénaturées obtenues par précipitation de l'échantillon de lait chauffé à pH 4.6, l'échantillon est analysé par électrophorèse capillaire pour obtenir les protéines sériques et les caséines. L'analyse du surnageant de précipitation permet de quantifier les protéines sériques solubles.

Parallèlement, sur ces mêmes échantillons, sont réalisées les étapes (a) à (g) du procédé selon l'invention.

Ainsi, ces échantillons sont transparisés (étape (a)) et sont effectuées d'une part, une mesure de diffusion conventionnelle (étape (b)) pour déterminer un indice PSD représentatif de la teneur en protéines sériques dénaturées et, d'autre part, une mesure de la fluorescence du tryptophane (étape (c)) pour déterminer un indice PT représentatif de la quantité totale de protéines.

D'autre part, les échantillons bruts sont traités pour isoler les protéines sériques solubles (étape (d)), les analyser grâce à un dosage par fluorescence du tryptophane présent dans le surnageant (étape (e)) pour déterminer un indice PSS représentatif de la teneur du surnageant en protéines sériques solubles, réaliser une mesure de diffusion dans le surnageant (étape (f)) pour déterminer un indice DP de la dénaturation des protéines et enfin déterminer un indice thermique global (indice FAST) par dosage, dans le surnageant, des sous-produits fluorescents issus de la réaction avancée de Maillard (étape (g)).

En pratique, on prend 500 µl de lait fromager qu'on dilue dans 10 ml d'eau, On mélange et on reprend 250 µl du lait dilué que l'on dilue dans 10 ml de tampon de transparisation. Après 5 minutes, le mélange est transparent et on peut mesurer la diffusion et la fluorescence au maximum d'émission du tryptophane (337 nm), après excitation à 280 nm. La transformation de la fluorescence du tryptophane en concentration de protéines sériques se fait par le biais d'un étalon de lait traité de la même manière à concentration connue de protéines totales (dosage par la méthode de référence Kjeldahl). On mesure également la diffusion à 280nm, après excitation à 280 nm.

Parallèlement, on reprend 1 ml de lait fromager et on le précipite avec 50 ml de tampon à pH 4,6. On filtre et on obtient une solution transparente qui ne contient que les protéines sériques solubles que l'on analyse à 337 nm, après excitation à 280 nm, pour en obtenir la concentration en protéines. On mesure également la diffusion à 280 nm et la concentration de produits de Maillard à 350 nm à l'excitation et 430 nm à l'émission, La transformation de la fluorescence du tryptophane en concentration de protéines sériques se fait par le biais d'un étalon de protéines sériques solubles à concentration connue (dosage par la méthode de référence Kjeldahl).

Une excellente régression est observée entre les mesures effectuées par électrophorèse capillaire et les cinq indices déterminés par les étapes (a) à (g) selon le procédé de l'invention d'une part, et la quantité de protéines sériques dénaturées, d'autre part. Le coefficient de corrélation est de 0.99 et l'erreur du modèle est de 0,49 g/L sur une distribution de teneurs entre 0 et 18 g/L.

Un premier modèle de régression multilinéaire est alors construit entre d'une part, la quantité de protéines dénaturées dosée par la méthode électrophorétique et d'autre part, une combinaison linéaire des diverses mesures réalisées selon les étapes (a) à (g) du procédé selon l'invention.

On obtient une régression de bonne qualité, avec une erreur de calibration donnée par l'indicateur RMSEC (Root Mean Square Error of Calibration), proche de l'erreur de la méthode de référence.

L'influence des différents indices sur l'erreur du résultat obtenu par le modèle ainsi construit et sur le coefficient de corrélation est indiquée dans le Tableau 1 ci-dessous.

Le modèle complet est basé sur les cinq indices donnés respectivement par les étapes (a) à (g) : indice PSD, indice PT, indice PSS, indice DP et indice FAST.

Le modèle correspondant aux laits et aux mesures de la figure 2 est défini par :

$$\text{Teneur en PSD (g/L)} = \text{Exp} \, (- \, 6{,}96683070 + 0{,}11696939 \, . \, \text{indice PSS} + 0{,}09522332 \, . \, \text{indice FAST} + 0{,}01943347 \, . \, \text{indice PT} + 0{,}00110096 \, . \, \text{indice DP} + 0{,}06505071 \, . \, \text{indice PSD}).$$

Bien entendu, pour d'autres composants de laits. les coefficients du modèle devraient être légèrement adaptés.

La valeur de l'erreur (RMSEC) et celle des coefficients de corrélation ($R^2$) sont données à la première ligne du Tableau 1.

Les lignes suivantes donnent ces mêmes valeurs lorsque le modèle n'est basé que sur quatre de ces cinq indices.

Tableau 1

| Modèle | RMSEC | $R^2$ |
|---|---|---|
| Complet | 0,49 | 0,99 |
| Sans indice PSS | 0,85 | 0,98 |
| Sans indice FAST | 0,48 | 0,99 |
| Sans indice PT | 0,88 | 0,98 |
| Sans indice DP | 0,87 | 0,98 |
| Sans indice PSD | 1,23 | 0,97 |

Il montre que l'indice FAST relatif à la charge thermique a une influence non significative sur le modèle.

Le modèle peut donc ne tenir compte que des indices obtenus par les étapes (a) à (f) du procédé, c'est-à-dire des indices PSS, PT, DP et PSD.

Le Tableau 1 montre que c'est l'indice PSD qui joue un rôle prépondérant dans ce premier modèle, même si les autres indices apportent une amélioration significative dans la qualité du modèle et donc dans la précision de la prédiction.

Comme indiqué précédemment, il est intéressant de déterminer le ratio entre les protéines sériques dénaturées et les caséines, indicateur qui est utile pour les industriels. Le ratio est, en effet, un bon prédicteur de la qualité technologique du lait fromager (temps de coagulation, vitesse d'égouttage et texture du caillé) et du rendement fromager.

Par ailleurs, les étapes (a) à (e) permettent d'estimer la teneur en caséines, qui correspond à la quantité totale de protéines (étape (c)) à laquelle sont soustraites la quantité de protéines sériques dénaturées (étapes (a) et (b)) et la quantité de protéines sériques non dénaturées (étapes (d) et (e)).

On comprend donc que les étapes (a) à (e) seront utiles pour estimer ce ratio.

La figure 3 est un graphique illustrant la régression entre la teneur en protéines sériques dénaturées (PSD) en pourcentage par rapport à la teneur en caséines, mesurée par électrophorèse capillaire et prédite par les étapes (a) à (g) du procédé selon l'invention,

Les mesures par électrophorèse capillaire et les étapes (a) à (f) du procédé selon l'invention ont été réalisées sur des échantillons fournis par des partenaires industriels et correspondant à des laits standardisés fromagers, comprenant du lait écrémé, du rétentat de microfiltration enrichi en caséines, éventuellement du rétentat de sérum chauffé pour permettre sa dénaturation, de la matière grasse laitière, et enfin un mélange de sels minéraux, notamment du calcium. Il s'agit des mêmes laits que ceux utilisés pour les mesures correspondant à la figure 2.

Ces mesures par électrophorèse capillaire et les étapes (a) à (g) sont réalisées comme expliqué précédemment et ne seront pas de nouveau décrites en détail.

La figure 3 montre qu'une excellente régression est observée entre les mesures effectuées par électrophorèse capillaire et les cinq indices déterminés par les étapes (a) à (g) selon le procédé de l'invention d'une part, et le ratio protéines sériques dénaturées (PSD)/caséines, d'autre part.

Le coefficient de corrélation $R^2$ est de 0,98 et l'erreur du modèle est d'environ 3%.

Un deuxième modèle de régression multilinéaire est alors construit entre d'une part, le ratio PSD/caséines dosé par la méthode électrophorétique et d'autre part, les différents indices obtenus lors des étapes (a) à (g) du procédé selon l'invention.

Le modèle correspondant aux faits et aux mesures de la figure 3 est défini par :

$$\text{Ratio PSD/Caséines} = \text{Exp} (-123{,}151918 + 1{,}60789852 \cdot \text{indice PT} + 0{,}07233199 \cdot \text{indice DP} - 6{,}43110855 \cdot \text{indice PSD} - 0{,}81489076 \cdot \text{indice PSS} - 0{,}02069923 \cdot \text{indice FAST}).$$

Bien entendu, pour d'autres compositions de laits, les coefficients du modèle devraient être légèrement adaptés.

On obtient une régression de bonne qualité avec une erreur de calibration donnée par l'indicateur RMSEC, proche de l'erreur de la méthode de référence.

L'influence des différents indices sur l'erreur du résultat obtenu par le modèle ainsi construit et sur le coefficient de corrélation est indiquée dans le Tableau 2 ci-dessous :

Tableau 2

| | RMSEC | $R^2$ |
|---|---|---|
| Toutes les variables | 0,68 | 0,98 |
| Sans indice PSS | 1,33 | 0,91 |
| Sans indice FAST | 1,09 | 0,94 |
| Sans Indice PT | 1,25 | 0,92 |
| Sans indice DP | 1,53 | 0,89 |
| Sans Indice PSD | 1,32 | 0,91 |

Ce Tableau 2 montre que tous les indices sont significatifs, mais que l'indice FAST apporte la contribution la moins importante au modèle. Cependant, if permet de faire baisser l'erreur du modèle de 1,09 à 0,68 et d'augmenter la valeur de $R^2$ de 0,94 à 0,98.

En pratique, le modèle peut ne tenir compte que des indices obtenus par les étapes (a) à (f) du procédé, c'est-à-dire les indices PSS, indices PT, DP et PSD.

Le Tableau 2 montre que c'est l'indice DP qui joue un rôle prépondérant dans ce deuxième modèle, suivi de l'indice PSD et PSS.

Ainsi, l'invention permet d'estimer la teneur en protéines sériques dénaturées dans un lait, que celui-ci comporte ou pas des matières grasses. Elle permet également d'estimer le ratio protéines sériques dénaturées / caséines dans un lait, quelle que soit sa nature.

Par ailleurs, les procédés selon l'invention permettent d'estimer ces grandeurs par des étapes dont la mise en œuvre est aisée, rapide et dont les coûts sont faibles par rapport aux procédés connus.

A titre d'exemple, une analyse d'un lait réalisée par électrophorèse capillaire nécessite de transmettre les échantillons à un laboratoire extérieur et d'attendre plusieurs jours pour obtenir le résultat et elle entraîne des coûts de plusieurs centaines d'euros.

Une analyse réalisée par un procédé selon l'invention peut être effectuée sur place, les résultats sont donc connus immédiatement (5 minutes) et les coûts sont de l'ordre de 15 euros.

Par ailleurs, contrairement aux procédés connus qui sont des méthodes de laboratoire, les procédés selon l'invention sont des procédés industriels.

## Revendications

1. Procédé d'estimation des protéines sériques dénaturées dans un lait ayant subi un traitement thermique, le procédé comportant les étapes successives consistant à :

   (a) rendre transparent un échantillon dudit lait à analyser,
   (b) mesurer la diffusion dans ledit échantillon transparisé pour en déduire un premier indice PSD représentatif de la teneur en protéines sériques dénaturées.

2. Procédé selon la revendication 1, comprenant, après l'étape (a), les étapes complémentaires suivantes consistant à :

   (c) déterminer un deuxième indice PT représentatif de la quantité totale de protéines dans l'échantillon transparisé à l'étape (a),
   (d) à provoquer la précipitation des caséines et des protéines sériques dénaturées dans un autre échantillon dudit lait, de manière à obtenir un précipité et un surnageant,
   (e) déterminer un troisième indice PSS représentatif de la teneur dudit surnageant en protéines sériques solubles,
   (f) déterminer un quatrième indice DP représentatif de la dénaturation des protéines dans ledit surnageant, et
   (h) estimer la teneur en protéines sériques dénaturées à partir desdits premier, deuxième, troisième et quatrième indices.

3. Procédé d'estimation du ratio protéines sériques dénaturées / caséines dans un lait ayant subi un traitement thermique, le procédé comportant un procédé d'estimation des protéines sériques dénaturées dans un lait ayant subi un traitement thermique selon la revendication 1, ledit procédé d'estimation du ratio protéines sériques dénaturées / caséines comportant en outre les étapes consistant à :

   (c) déterminer un deuxième indice PT représentatif de la quantité totale de protéines dans l'échantillon transparisé à l'étape (a),
   (d) à provoquer la précipitation des caséines et des protéines sériques dénaturées dans un autre échantillon dudit lait, de manière à obtenir un précipité et un surnageant,
   (e) déterminer un troisième indice PSS représentatif de la teneur dudit surnageant en protéines sériques solubles,
   (f) déterminer un quatrième indice DP représentatif de la dénaturation des protéines dans ledit surnageant et
   (i) estimer le ratio protéines sériques dénaturées/caséines à partir desdits premier, deuxième, troisième et quatrième indices.

4. Procédé selon la revendication 2 ou 3 comprenant, après l'étape (d), une étape complémentaire (g) consistant à déterminer un cinquième indice FAST représentatif d'un niveau de traitement thermique subi par ledit échantillon.

5. Procédé selon l'une des revendications 1 à 4 dans lequel au moins une desdites étapes (a) à (g) met en oeuvre une mesure optique pour déterminer l'indice correspondant.

6. Procédé selon l'une des revendications 2 à 5, dans lequel, dans l'étape (c), le deuxième indice PT est déterminé par une mesure de fluorescence du tryptophane dans l'échantillon transparisé.

7. Procédé selon l'une des revendications 2 à 6, dans lequel, dans l'étape (d), la précipitation des protéines est obtenue en ajoutant à un autre échantillon dudit lait un tampon de force ionique et de pH appropriés.

8. Procédé selon l'une des revendications 2 à 7, dans lequel, dans l'étape (e), le troisième indice PSS est obtenu par une mesure de la fluorescence du tryptophane dans le surnageant transparent obtenu à l'étape (d).

9. Procédé selon l'une des revendications 2 à 8, dans lequel, dans l'étape (f), le quatrième indice DP est obtenu par une mesure de la diffusion dans le surnageant transparent obtenu à l'étape (d).

10. Procédé selon l'une des revendications 3 à 9, dans lequel le cinquième indice FAST est représentatif d'une teneur du surnageant obtenu à l'étape (d) en produits de Maillard.

11. Procédé selon la revendication 10, dans lequel, dans l'étape (g), le cinquième indice FAST est obtenu par une mesure de fluorescence desdits produits de Maillard.

12. Procédé selon la revendication 2, dans lequel, l'étape (h) est mise en oeuvre par régression multilinéaire.

13. Procédé selon la revendication 3, dans lequel, l'étape (i), est mise en œuvre par régression multilinéaire.


**Patentansprüche**

1. Verfahren zur Schätzung der denaturierten Serumproteine in einer wärmebehandelten Milch, wobei das Verfahren die folgenden aufeinanderfolgenden Schritte umfasst:

   (a) Transparentmachen einer Probe der besagten zu analysierenden Milch,
   (b) Messen der Diffusion in der besagten transparent gemachten Probe, um daraus einen ersten PSD-Index abzuleiten, der für den Gehalt an denaturierten Serumproteinen repräsentativ ist.

2. Verfahren nach Anspruch 1, das nach Schritt (a) die folgenden zusätzlichen Schritte umfasst:

   (c) Bestimmen eines zweiten PT-Index, der für die Gesamtproteinmenge in der in Schritt (a) transparent gemachten Probe repräsentativ ist,
   (d) Herbeiführen der Fällung der Kaseine und der denaturierten Serumproteine in einer anderen Probe der besagten Milch, um einen Niederschlag und einen Überstand zu erhalten,

(e) Bestimmen eines dritten PSS-Index, der für den Gehalt des besagten Überstands an löslichen Serumproteinen repräsentativ ist,

(f) Bestimmen eines vierten DP-Index, der für die Denaturierung der Proteine in dem Überstand repräsentativ ist, und

(h) Schätzen des Gehalts an denaturierten Serumproteinen ausgehend von dem besagten ersten, zweiten, dritten und vierten Index.

3. Verfahren zur Schätzung des Verhältnisses von denaturierten Serumproteinen zu Kaseinen in einer wärmebehandelten Milch, wobei das Verfahren ein Verfahren zur Schätzung der denaturierten Serumproteine in einer wärmebehandelten Milch nach Anspruch 1 umfasst, wobei das besagte Verfahren zur Schätzung des Verhältnisses von denaturierten Serumproteinen zu Kaseinen ferner die folgenden Schritte umfasst:

(c) Bestimmen eines zweiten PT-Index, der für die Gesamtproteinmenge in der in Schritt (a) transparent gemachten Probe repräsentativ ist,

(d) Herbeiführen der Fällung der Kaseine und der denaturierten Serumproteine in einer anderen Probe der besagten Milch, um einen Niederschlag und einen Überstand zu erhalten,

(e) Bestimmen eines dritten PSS-Index, der für den Gehalt des besagten Überstands an löslichen Serumproteinen repräsentativ ist,

(f) Bestimmen eines vierten DP-Index, der für die Denaturierung der Proteine in dem besagten Überstand repräsentativ ist, und

(i) Schätzen des Verhältnisses von denaturierten Serumproteinen zu Kaseinen ausgehend von dem besagten ersten, zweiten, dritten und vierten Index.

4. Verfahren nach Anspruch 2 oder 3, das nach Schritt (d) einen zusätzlichen Schritt (g) umfasst, der in dem Bestimmen eines fünften FAST-Index besteht, der für ein Niveau der Wärmebehandlung repräsentativ ist, dem die besagte Probe unterzogen wird.

5. Verfahren nach einem der Ansprüche 1 bis 4, worin mindestens einer der besagten Schritte (a) bis (g) eine optische Messung umsetzt, um den entsprechenden Index zu bestimmen.

6. Verfahren nach einem der Ansprüche 2 bis 5, worin in Schritt (c) der zweite PT-Index durch eine Messung der Fluoreszenz des Tryptophans in der transparent gemachten Probe bestimmt wird.

7. Verfahren nach einem der Ansprüche 2 bis 6, worin in Schritt (d) die Fällung der Proteine durch Zugabe eines Puffers mit geeigneter Ionenstärke und geeignetem pH-Wert zu einer anderen Probe der besagten Milch erhalten wird.

8. Verfahren nach einem der Ansprüche 2 bis 7, worin in Schritt (e) der dritte PSS-Index durch eine Messung der Fluoreszenz des Tryptophans in dem in Schritt (d) erhaltenen transparenten Überstand erhalten wird.

9. Verfahren nach einem der Ansprüche 2 bis 8, worin in Schritt (f) der vierte Index DP durch eine Messung der Diffusion in dem in Schritt (d) erhaltenen transparenten Überstand erhalten wird.

10. Verfahren nach einem der Ansprüche 3 bis 9, worin der fünfte FAST-INDEX repräsentativ für einen Gehalt des in Schritt (d) erhaltenen Überstands an Maillard-Produkten ist.

11. Verfahren nach Anspruch 10, worin in Schritt (g) der fünfte FAST-Index durch eine Messung der Fluoreszenz der besagten Maillard-Produkte erhalten wird.

12. Verfahren nach Anspruch 2, worin Schritt (h) durch multilineare Regression umgesetzt wird.

13. Verfahren nach Anspruch 3, worin Schritt (i) durch multilineare Regression umgesetzt wird.

**Claims**

1. A method to estimate denatured serum proteins in milk having undergone heat treatment, the method comprising the successive steps of:

(a) rendering transparent a sample of said milk to be analysed;
(b) measuring scattering in said optically cleared sample to deduce a first index, DSP, representing the content of denatured serum proteins.

2. The method according to claim 1 comprising, after step (a), the following additional steps of:

(c) determining a second index, TP, representing the total quantity of proteins in the sample optically cleared at step (a);
(d) causing precipitation of the caseins and denatured serum proteins in another sample of said milk, to obtain a precipitate and a supernatant;
(e) determining a third index, SSP, representing the content of soluble serum proteins in said supernatant;
(f) determining a fourth index, PD, representing protein denaturation in said supernatant; and
(h) estimating the content of denatured serum proteins from said first, second, third and fourth indices.

3. A method to estimate the denatured serum protein / casein ratio in milk that has been subjected to heat treatment, the method comprising the method to estimate denatured serum proteins in milk having undergone heat treatment according to claim 1, the method to estimate the denatured serum protein / casein ratio comprising the steps of consisting of:

(c) determining a second index, TP, representing the total quantity of proteins in the sample optically cleared at step (a),
(d) causing precipitation of the caseins and denatured serum proteins in another sample of said milk, to obtain a precipitate and a supernatant;
(e) determining a third index, SSP, representing the content of soluble serum proteins in said supernatant;
(f) determining a fourth index, PD, representing protein denaturation in said supernatant; and
(i) estimating the denatured serum protein / casein ratio from said first, second, third and fourth indices.

4. The method according to claim 2 or 3 comprising, after step (d), an additional step (g) whereby a fifth index, FAST, is determined representing the intensity of heat treatment undergone by said sample.

5. The method according to one of claims 1 to 4, wherein at least one of said steps (a) to (g) uses optical measurement to determine the corresponding index.

6. The method according to one of claims 2 to 5, wherein at step (c) the second index TP is determined by measuring the fluorescence of tryptophan in the optically cleared sample.

7. The method according to one of claims 2 to 6, wherein at step (d) precipitation of the proteins is obtained by adding a buffer of suitable ionic strength and pH to another sample of said milk.

8. The method according to one of claims 2 to 7, wherein at step (e) the third index SSP is obtained by measuring the fluorescence of tryptophan in the transparent supernatant obtained at step (d).

9. The method according to one of claims 2 to 8, wherein at step (f) the fourth index PD is obtained by measuring scattering in the transparent supernatant obtained at step (d).

10. The method according to one of claims 3 to 9, wherein the fifth index FAST represents the Maillard product content of the supernatant obtained at step (d).

11. The method according to claim 10, wherein at step (g) the fifth index FAST is obtained by measuring the fluorescence of said Maillard products.

12. The method according to claim 2, wherein step (h) is implemented by multilinear regression.

13. The method according to claim 3, wherein step (i) is implemented by multilinear regression.

Figure 1

Figure 2

Figure 3

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3841834 A

- EP 0922221 A

**Littérature non-brevet citée dans la description**

- **V. BONFATTI et al.** *Validation of a new reversed-phase high-performance liquid chromatography method for separation and quantification of bovine milk protein genetic variants*
- **G. BOBE et al.** *Séparation and quantification of bovine milk proteins by reversed-phase high-performance liquid chrornatography*
- **L. RECIO et al.** *Détermination of denatured serum proteins in the casein fraction of heai-treated milk by capillary zone efectrophoresis*
- **BOGOMOLOV et al.** *Food Chemistry,* 2012, vol. 134, 412-418

- **LAMB et al.** *J. Dairy sci.,* vol. 96, 1356-1365
- **DE FOX et al.** *Journal of Dairy Science,* Avril 1963, vol. 46 (4), 302-309
- **C.F. BOHREN ; D,R. HUFFMAN.** Absorption and scattering of light by small particles. John Wiley & Sons, 1983
- **H. C. VAN DE HULST.** Light scattering by small particles. John Wiley and Sons), London (Chapman and Hall), 1957
- **AKIRA ISHIMARU.** Wave Propagation and Scattering in Random Media. Wiley-IEEE Press, Janvier 1999